Europäisches Patentamt

European Patent Office

Office européen des brevets

(11) Publication number: **0 282 164**
**A2**

## (12) EUROPEAN PATENT APPLICATION

(21) Application number: **88301083.7**

(22) Date of filing: **09.02.88**

(51) Int. Cl.⁴ **C12N 5/00**

The title of the invention has been amended (Guidelines for Examination in the EPO, A-III, 7.3).

(30) Priority: **09.02.87 JP 26411/87**

(43) Date of publication of application:
**14.09.88 Bulletin 88/37**

(84) Designated Contracting States:
**BE DE FR GB IT LU NL**

(71) Applicant: **MITSUI PETROCHEMICAL INDUSTRIES, LTD.**
**2-5, Kasumigaseki 3-chome Chiyoda-ku Tokyo 100(JP)**

(72) Inventor: **Motoyama, Yoshio**
**4-10, Wakichou Waki 2-chome**
**Kuga-gun Yamaguchi 740(JP)**
Inventor: **Kitani, Shigekazu**
**4-9, Wakichou Waki 2-chome**
**Kuga-gun Yamaguchi 740(JP)**
Inventor: **Matubara, Kouichi**
**67-11, Muronokicho 4-chome**
**Iwakuni-shi Yamaguchi 740(JP)**

(74) Representative: **Myerscough, Philip Boyd et al**
**J.A.Kemp & Co. 14, South Square Gray's Inn**
**London, WC1R 5EU(GB)**

(54) **Method for plant tissue culture.**

(57) A method for the tissue culture of a plant in a liquid medium, in which a phytohormone is fed to the medium intermittently under a defined condition to enable a stable culture for a long period with a high growth rate of the cell.

EP 0 282 164 A2

## METHOD FOR THE TISSUE CULTURE OF A PLANT

### BACKGROUND OF THE INVENTION

#### 1. Field of the Invention

The present invention relates to a method for the tissue culture of a plant. More particularly, the present invention relates to a method for the tissue culture of a plant in a liquid medium, in which a phytohormone is fed to the medium intermittently under a defined condition to enable a stable culture for a long period with a high growth rate of the cell.

#### 2. Prior art

No conventional method for the tissue culture include a procedure in which the concentration of the component in the medium such as a phytohormone is appropriately adjusted by additionally feeding it after the start of the culture using a medium containing the phytohormone at a defined concentration.

The medium component such as a phytohormone is consumed by the cells after the start of the culture and the concentration may be gradually lowered possibly to an unfavorable range for some components to continue the culture. Such a conventional method of the batch culture gives a low growth rate of the cell and also gives a low growth stability of the cells.

On the other hand, a method for the culture has been known in which the culture liquid is discharged out of the culture region together with the cultured product to refresh the medium. For example,"Fermentation Technology (Hakko Kogaku)" 61, 117 - 128 (1983) describes a method for the continuous culture of tobbaco plant cell in which a liquid medium containing a phytohormone, 2,4-D-(2,4-dichlorophenoxyacetic acid) is renewed continuously. In this case, an almost constant concentration of the phytohormone is thought to be present in the medium during the culture period. As a method for the culture under renewal of the medium, Japanese Patent Publication No. 36915 of 1986 shows a method for the high concentration culture of suspended cells in which the culture suspended liquor obtained by the sedimentation of the cells in the medium liquor is discharged out of the culture vessel and at the same time a fresh medium is supplied. However, the method for the culture shown in these conventional methods have an insufficient growth rate and shows an unsatisfactory growth stability of the culture cells and thus the production of the secondary metabolites including alkaloids and pigments is not enough to leave room for improvement.

#### 3. Problems to be solved by the invention

From these backgrounds, we. inventors. have investigated the method for the culture in which the growth rate of the cells is higher than the conventional methods and which gives an excellent growth stability and produces the secondary metabolites efficiently in a high yield and recognized that the concentration of the phytohormone shall not be maintained necessarily during the culture period and it shall be rather changed appropriately in accordance with the growth of the cells. We have completed the invention related to the method for the tissue culture which can accomplish the above mentioned object based on these knowledges.

#### Summary of the invention

Thus, according to the present invention, a method for the tissue culture of a plant using a liquid medium is provided in which a phytohormone is fed to the medium intermittently so that the following conditions (a) and (b) are satisfied:

(a) The feeding interval, D (time), of the phytohormone is set at a value 0.5 to 5 times as long as the doubling time of the cell.

(b) An amount of the phytohormone satisfying the equation (I) is fed to the medium.

$$10^{-12} < \frac{A}{D \cdot W} < 10^{-5} \quad \text{(I)}$$

where A is the amount of the phytohormone fed intermittently to the medium (mol), W is the fresh weight of the cells present in the culture region (g) and D is the feeding interval of the phytohormone (day).

#### DESCRIPTION OF THE PREFERRED EMBODIMENT

The plants for the application of the method for the tissue culture of a plant according to the present invention are not especially limited but the method of the present invention can be applied fundamentally to a plant to which a conventional tissue culture can be applied. Such plants include, for example, the plants of Duboisia genus such as Duboisia myoporoides, Duboisia leichhardtii, etc.,

the plants of Datura genus such as Datura tatula, Datura arborea, Datura stramonium, etc., the plants of Scopolia genus such as Scopolia japonica, etc., the plants of Solanaceae family including the plants of Hyos genus such as Hyosoyamus niger and the plants of Atropa genus such as Atropa belladonna, etc., the plants of Coptis genus such as Coptis japonica Makino, C. japonika Makino var. japonika, C. japonika Makino var. major Satake, C. quinquefolia Miq. and C. trifolia Salisb., etc., the plants of Thalictrum genus such as Thalictrum minus L. var hypolecum Miq., etc., the plants of Ranunculaceae family including the plants of Thalintrige genus and Hydrastis genus, etc., the plants of Papaveraceae family such as Papaver somniferum, etc., the plants of Legminosae family such as Glycyrrhiza uralensis, the plants of Umbelliferae family such as carrot, Bupleurum falcatum, etc., the plants of Polygonaceae family such as Rheum palmatum, the plants of Apocynaceae family such as Rauwolfia serpentina, Madagascar periwinkle, etc., the plants of Solanaceae family such as Nicotiana tabacum, the plants of Borraginaceae family such as Lithospermum erythrorhizon, the plants of Labiatae family such as Perilla frutesceus var. acuta, Mentha arvensis var. piperasceus, etc., the plants of Rubiaceae family such as Coffea arabica, the plants of Scrophulariaceae family such as Digitalis purpurea.

According to the present invention, the tissue (including the cells and the organs) of said plant is carried out using a liquid medium and the well known media which have been used for the tissue culture of a plant can be used as the liquid medium of the present invention. Said medium contains typically inorganic components, carbon sources and phytohormones as the essential ingredients and also contains vitamines additionally and further contains, if required, amino acids. The inorganic components of said medium include inorganic salts containing such elements as nitrogen, phosphor, potassium, sodium, calcium, magnesium, sulfur, iron, manganese, zinc, boron, molybdenum, chlorine, iodine and cobalt, etc. and exemplified by the compounds such as potassium nitrate, sodium nitrate, ammonium nitrate, ammonium chloride, potassium chloride, calcium chloride, potassium monohydrogen phosphate, sodium dihydrogen phosphate, magnesium sulfate, magnesium chloride, sodium sulfate, ferrous sulfate, ferric sulfate, manganese sulfate, copper sulfate, sodium molybdate, molybdenum oxide, potassium iodide, zinc sulfate, boric acid, cobalt chloride, etc..

The carbon sources of said medium include, for example, a carbohydrate such as sucrose and a derivative thereof, an organic acid such as a fatty acid and a primary alcohol such as ethanol, etc..

The phytohormones of said medium include, for example, an auxin such as indol acetic acid (IAA), naphthalene acetic acid (NAA), p-chlorophenoxy isobutyric acid and 2,4-dichlorophenoxy-acetic acid (2,4-D), etc. and a cytokinin such as kinetin, zeatin and benzyl adenine, etc..

The vitamines of said medium include, for example, biotin, thiamin (vitamin $B_1$), pyridoxine (vitamin $B_6$), pyridoxal, pyrodoxamine, calcium pantothenate, ascorbic acid (vitamin C), inositol, nicotinic acid, nicotinamide and riboflavin (vitamin $B_2$), etc..

The amino acids of said medium include, for example, glycine, alanine, glutamic acid, cysteine, tyrosine and lysine, etc..

It is preferred that the liquid medium used for the present invention contains approx. 0.1 $\mu$M to approx. 100mM of said inorganic component, approx. 1 g/$\ell$ to approx. 100 g/$\ell$ of said carbon source, approx. 0.1 mg/$\ell$ to approx. 150 mg/$\ell$ of said vitamine and 0 to approx. 1000 mg/$\ell$ of said amino acid. Although a defined amount of a phytohormone shall be fed intermittently to the medium under a defined condition according to the present invention, the concentration of the phytohormone in the medium immediately before the intermittent addition of the phytohormone may be usually zero (below the detectable limit) and, if required, it may be in the range of 0 to $10^{-11}$ M. And, an amoint of the phytohormone defined by the equation (I) mentioned below under a defined condition mentioned below shall be fed intermittently to the medium so that the concentration of the phytohormone in the medium immediately after the intermittent addition of the phytohormone is in the range of $10^{-4}$ to $10^{-10}$ M, preferably $10^{-5}$ to $10^{-8}$ M.

The medium used for the present invention may be typically a well known medium used for the tissue culture of a plant, for example, a medium prepared by a procedure in which the above mentioned carbon source is added to a medium such as Murashige & Skoog's ('62) medium, Linsmaier & Skoog's (RM-1965) medium, White's ('63) medium, Gamborg's B-5 medium and Mitsui's M-9 medium and further the phytohormone, the vitamine and the amino acid are added if required. It is preferred that especially a medium prepared using Linsmaier & Skoog's medium or Murashige & Skoog's medium among them is used. The compositions of the above-mentioned known media are disclosed in Takeuchi, Nakajima and Furutani "New Plant Tissue Culture" p386 - 391 (Asakura Shoten, 1979).

The tissue culture of said plant using said liquid medium according to the present invention is carried out under the condition mentioned below characterizing the present invention. Said condition will be described below in detail.

The cell concentration in the liquid medium in the method for the tissue culture of a plant according to the present invention may be at one's option and it is preferred that a culture using a cell concentration of 100 to 500 g/ℓ of the liquid medium, preferably 200 to 400 g/ℓ, is carried out as such a condition gives a high yield of the cells per unit volume of the culture vessel and thus results in an efficient culture.

A cell concentration higher than 500 g/ℓ usually causes a high slurry concentration and provides an insufficient stirring or admixture of the culture liquid to prevent an effective oxygen supply and thus causes the necrosis and the damage of the cells during the culture and makes the culture difficult. Because such as a high cell concentration as above 500 g/ℓ causes difficulty in the culture, it is preferred that the culture is carried out at a cell concentration not higher than 500 g/ℓ. As the number of the cells increases by the growth during the culture period, the cell concentration in the culture region can be maintained at an appropriate level by discharging the cells out of the culture region in an appropriate amount by an appropriate method during the culture period. The weight of the cells used for the calculation of the cell concentration is the fresh weight measured under a wet condition. The fresh weight can be usually determined by the following procedure: The tissue cultured product is placed on a filter cloth laid on a porcelain funnel and filtered using a water-jet pump for 5 min. to remove the culture liquid other than the cells and the weight of the remained cells is measured. The cells obtained by such a method contains usually 80 to 95 weight % of water although varied by the sort of the plant.

The tissue pieces and the cells of a plant are cultured in the tissue culture according to the present invention. Said tissue pieces include, for example, stalk-tops, stalks, leaves, flowers, seeds and roots. Calluses derived from the tissue pieces and the cultured cells can be also used as the raw materials for the tissue culture.

Although the method for the tissue culture according to the present invention can be carried out either by a batch culture or by a continuous culture, it is preferred to use a continuous culture. In the case of the continuous culture, the tissue culture can be carried out by a procedure in which a liquid medium containing a nutrition substrate adjusted to a defined concentration is fed to the culture region continuously or discontinuously, while the culture liquid is discharged from the other end of the culture region continuously or discontinuously and thus the tissue culture is carried out under renewal of the medium liquid in the culture region. The renewal of the culture liquid may be continuous or discontinuous.

The nutrition substrate means a medium component in the liquid medium used according to the present invention mentioned above and includes an inorganic component, a carbon source, a phytohormone, a vitamin and an amino acid.

The culture liquid containing a nutrition substrate adjusted to a defined concentration is a liquid medium containing approx. 0.1 $\mu$M to approx. 100 mM of an inorganic component, approx. 1 to approx. 100 g/ℓ of a carbon source, 0 to approx. 150 mg/ℓ of a vitamin and 0 to approx. 1000 mg/ℓ of an amino acid as mentioned above. In the case of the continuous culture under renewal of the liquid medium, it is preferred that the concentration of the phytohormone in the liquid medium fed to the culture region is usually zero (below the detectable limit). However, it may be, if required, in the range of 0 to $10^{-11}$ M. The concentration of the phytohormone in the culture region immediately after the intermittent addition of the phytohormone to the culture region is usually in the range of $10^{-4}$ to $10^{-10}$M, preferably $10^{-5}$ to $10^{-8}$ M as mentioned above. Although it is usually preferred to use a fresh liquid medium as the liquid medium containing the medium components adjusted to the above-mentioned range of the concentration fed to the culture region according to the present invention, a procedure may be used in which the concentration of the nutrition substrate in the medium discharged from the culture region is adjusted and then the medium is recycled for use. In this case, it is preferred to remove appropriately the waste product of the metabolism of the cells in the culture liquid.

In the case the tissue culture is carried out under renewal of the culture liquid according to the present invention, it is preferred that the following equation is satisfied:

$$\mu \leq F/V < 20 \, \mu$$

where V is the amount of the culture liquid in the culture vessel (culture region) (ℓ), F is the amount of the liquid medium fed to the culture vessel (ℓ/day), F/V is the renewal rate of the medium and $\mu$ is the relative growth rate of the tissue culture product (day$^{-1}$). Here, the relative growth rate $\mu$ (day$^{-1}$) is defined as follows.

Thus, in the case the cultured cells are not discharged out of the culture region, the relative growth rate $\mu$ is defined by the following equation:

$$\mu = \frac{\ell n \dfrac{xt}{xo}}{t}$$

where xo is the amount of the cells at a time of to during the culture and xt is the amount of the cells after a time t (day).

In the case the grown cells are discharged out of the culture region while maintaining the amount of the cells at xo, the relative growth rate $\mu$ is defined by the following equation:

$$\mu = \frac{yt}{xo \cdot t}$$

where yt is the amount of the cells discharged out of the culture region after a time t (day).

$\mu$ has a dimension of (time$^{-1}$) and a higher $\mu$ value means a more rapid growth of the tissue culture product.

The tissue culture according to the present invention is usually carried out at a relative growth rate, $\mu$, in the range of 0.02 to 0.4 (day$^{-1}$), preferably 0.05 to 0.2 (day$^{-1}$), although varied by the sort of the plant. In the case a method of continuous culture is applied according to the present invention, it is preferred that the feeding amount F ($\ell$/day) of the liquid medium containing a nutrition substrate adjusted to a defined concentration when fed continuously or discontinuously to the culture region is set to satisfy the equation:

$\mu \leqq F/V < 20 \mu$,

as mentioned above. The renewal rate F/V of the medium in this case is usually in the range of 0.02 to 8 (day$^{-1}$), preferably 0.05 to 4 (day$^{-1}$).

In the case the culture liquid is discharged continuously or discontinuously from the other end of the culture region according to the present invention, the concentration of the cell in the culture vessel during the culture is maintained preferably at a concentration in the range of 100 to 500 (g/$\ell$). In this case, only the culture liquid may be discharged from the culture vessel and, if required, an appropriate amount of the cells may be discharged together with the culture liquid.

In the case the medium renewal rate (F/V) is smaller than the relative growth rate $\mu$, the concentration of the cell becomes too high and the concentration of the cell cannot be kept optimum. Hence, it is preferred that the culture liquid is discharged from the culture region together with an appropriate amount of the culture cell rather than the culture liquid only is discharged. For example, the culture liquid containing the cells can be discharged from the discharge tube equipped to the culture vessel and at the same time a liquid medium containing a nutrition substrate adjusted to the above-mentioned definite concentration is sent to the culture vessel to maintain the concentration of the cell in the culture vessel at an appropriate level during the culture.

In the case the continuous culture method is used in the present invention in which the medium renewal rate (F/V) is extremely larger than the relative growth rate 20 $\mu$, bad effects such as the melanism and the necrosis of the cells may be apt to occur usually. Thus, it is especially preferred that the tissue culture is carried out by controlling the medium renewal rate (F/V) within the range satisfying the equation:

$\mu \leqq F/V < 20 \mu$

The amount of the culture liquid discharged from the other end of the culture region continuously or discontinuously corresponds to the amount of the medium ($\ell$/day) fed to the culture region usually as mentioned above. However, it is not limited to this amount F and, for example, the discharge amount of the medium can be made somewhat larger than the value of F ($\ell$/day) in the case the culture is carried out under a condition satisfying the equation: F/V < $\mu$ mentioned above.

According to the present invention, the phytohormone is fed intermittently to the medium so that the following conditions (a) and (b) are satisfied as mentioned above:

(a) The feeding interval, D (hr), of the phytohormone is set at a value 0.5 to 5 times as long as the doubling time of the cells.

(b) An amount of the phytohormone satisfying the equation (I) is fed to the medium.

$$10^{-12} < \frac{A}{D \cdot W} < 10^{-6} \quad (I)$$

where A is the amount of the phytohormone fed intermittently to the medium (mol), W is the fresh weight of the cells present in the culture region (g) and D is the feeding interval of the phytohormone (day).

The feeding method of the phytohormone according to the present invention will be described in detail as follows. Although an amount (A) of the above-mentioned phytohormone satisfying the equation (I) in (b) is fed intermittently to the medium according to the present invention, the intermittent feeding is made by a procedure in which the intermittent time interval D for the feeding of the phytohormone is set at a value 0.5 to 5 times as long as the doubling time of the cell in the culture region as shown in (a). Here, the doubling time of the cell means the time required for doubling the amount of the growing cells in the culture region now in question and the typical doubling time is usually 2 to 20 days although varied by the sort of the plant and the culture condition. In the case the intermittent time interval D for feeding the phytohormone is shorter than 0.5 times of the doubling time of the cell, the melanism and the necrosis of the cells occur and the growth of the cells is highly prevented as the culture period is prolonged. In the case the phytohormone is fed so that D is not smaller than 5 times of the doubling time of the cells, the secondary metabolites may be discharged from the cells or a temporary growth inhibition of the cells may occur thought to be caused by the hormone abnormality immediately after the feeding as the amount of the phytohormone fed at a time becomes higher and thus a culture stable for a long period becomes difficult to be performed. As the above two conditions are unfavorable, the time interval for the intermittent feeding of the phytohormone is set at the range mentioned above.

The amount (A) of the phytohormone shown in the above equation (I) is the amount fed intermittently to the medium. However, the culture is carried out so that the concentration of the phytohormone in the medium immediately after the intermittent addition of the phytohormone is in the range of $10^{-5}$ to $10^{-8}$ M usually according to the present invention. Hence, the amount (A) of the phytohormone fed intermittently to the medium shall be set so that the amount (A) satisfies the equation (I) and at the same time the concentration of the phytohormone in the medium immediately after the intermittent addition of the phytohormone (the concentration based on the total amount of the phytohormone fed intermittently and that remained in the medium before the feeding) is in the range of $10^{-5}$ to $10^{-8}$ M mentioned above. Accordingly, the amount (A) of the phytohormone to be added shall be decided under a consideration of the phytohormone remained in the medium in addition to be limited to the equation (I). And, in the case a procedure according to the present invention is used in which the conditions (a) and (b) are satisfied at the time the concentration of the phytohormone remained in the medium becomes substantially zero and the phytohormone is fed intermittently to the medium so that the concentration of the phytohormone falls into the above-mentioned range, a normal cycle of the division and the hypertrophy of the cells is maintained and the growth of the cells and the production of the secondary metabolites can be continued for a long period to give a favorable result. A procedure can be used naturally if required according to the present invention in which the phytohormone is fed intermittently so that the conditions (a) and (b) are satisfied at any concentration not substantially zero in the range mentioned above.

In the case the value of $\dfrac{A}{D \cdot W}$

in the equation (I) is not larger than $10^{-12}$ according to the present invention (the amount of the phytohormone fed intermittently is small), the growth rate of the cells becomes low and the growth of the cells becomes insufficient to give an unfavorable result. In the case the value is not lower than $10^{-6}$, the concentration of the phytohormone in the medium becomes too high and the necrosis of the cells may occur and the production of the secondary metabolites becomes very low. Thus, the amount of the phytohormone fed intermittently according to the present invention is set within the above-mentioned range.

The method of the tissue culture according to the present invention can prepare efficiently in a high yield the secondary metabolites including the alkaloids such as berberine, atropine, scopolamine, etc. and the pigment components such as shikonin, puripurine, alizarin, etc..

The method for the tissue culture according to the present invention in which the phytohormone is fed intermittently to the medium under a defined condition can prepare efficiently in a high yield the secondary metabolites as it gives a higher growth rate of the cells than the conventional method and also gives an excellent growth stability of the cells.

The method of the present invention will be illustrated by Examples as follows.

## Example 1

The culture cells of Coptis japonica were cultured in a 2 liter aerated culture vessel using a Linsmaier & Skoog's medium. The volume of the liquid medium in the culture vessel during the culture was 1.8 liters. A liquid medium containing a nutrition substrate adjusted to a defined concentration was fed continuously (540 ml/day) to the culture vessel so that the concentration of the cells of Coptis japonica becomes to be 300 g/l as the fresh weight and at the same time a hormone component ($9 \times 10^{-5}$ mol of naphthalene acetic acid and $9 \times 10^{-8}$ mol of benzyl adenine) was added at 4 day's interval (equal to the doubling time) while discharging the medium and the grown cells from the other end of the culture vessel to perform the culture.

After the continuous culture was carried out for 60 days, the relative growth rate of the cells of Coptis japonica was 0.18 ($day^{-1}$) and the berberine content in the cells of Coptis japonica was 5.0%.

## Example 2

The procedure of Example 1 was repeated except that the liquid medium containing a nutrition substrate adjusted to a defined concentration was continuously fed to the culture vessel (540 ml/day) and at the same time a hormone compo-

nent ($3 \times 10^{-4}$ mol of naphthalene acetic acid and $3 \times 10^{-7}$ mol of benzyl adenine) was added at 12 day's interval (3 times of the doubling time).

The relative growth rate of the cells of Coptis japonica was 0.17 (day$^{-1}$) and the berberine content in the cells of Coptis japonica was 4.9%.

## Example 3

The procedure of Example 1 was repeated except that the liquid medium containing a nutrition substrate adjusted to a defined concentration was fed continuously to the culture vessel (540 mℓ/day) and at the same time a hormone component ($4 \times 10^{-4}$ mol of naphthalene acetic acid and $4 \times 10^{-7}$ mol of benzyl adenine) was added at 18 day's interval. The relative growth rate of the cells of Coptis japonica was 0.16 (day$^{-1}$) and the berberine content in the cells of Coptis japonica was 4.7%.

## Comparative Example 1

The procedure of Example 1 was repeated except that the liquid medium containing a nutrition substrate adjusted to a defined concentration was fed continuously to the culture vessel (540 mℓ/day) and at the same time a hormone component ($5 \times 10^{-4}$ mol of naphthalene acetic acid and $5 \times 10^{-7}$ mol of benzyl adenine) was added at 21 day's interval (5.3 times of the doubling time). The relative growth rate of the cells of Coptis japonica and the berberine content in the cells tended to be lowered gradually as the cultured period was prolonged. After 60 days culture, the relative growth rate and the berberine content were resp. 0.08 (day$^{-1}$) and 2.0%.

## Comparative Example 2

The procedure of Example 1 was repeated except that the liquid medium containing a nutrition substrate adjusted to a defined concentration was fed continuously to the culture vessel (540 mℓ/day) and at the same time a hormone component ($5 \times 10^{-2}$ mol of naphthalene acetic acid and $5 \times 10^{-5}$ mol of benzyl adenine) was added at 4 days interval (equal to the doubling time). Melanism (necrosis) of the cells occurred and the relative growth rate and the berberine content of the cells were resp. 0.05 (day$^{-1}$) and 1.5% after 30 days culture.

## Comparative Example 3

The procedure of Example 1 was repeated except that the liquid medium containing a nutrition substrate adjusted to a defined concentration was fed continuously to the culture vessel (540 mℓ/day) and at the same time a hormone component ($10^{-5}$ mol of naphthalene acetic acid and $10^{-8}$ mol of benzyl adenine) was added at 8 hours interval (0.08 times of the doubling time). After approx. 7 days culture, melanism of the calluses began and the relative growth rate and the berberine content after 30 days culture were resp. 0.10 (day$^{-1}$) and 2.5%.

## Comparative Example 4

The procedure of Example 1 was repeated except that the liquid medium containing a nutrition substrate adjusted to a defined concentration was fed continuously to the medium (540 mℓ/day) and at the same time a hormone component ($10^{-9}$ mol of naphthalene acetic acid and $10^{-12}$ mol of benzyl adenine) was added at 4 days interval (equal to the doubling time). Melanism and necrosis didn't occur but the growth of the cells was poor and the relative growth rate was 0.11 (day$^{-1}$) and the berberine content was 5%.

Table 1 Tissue culture of Coptis japonica

| | D/doubling time of the cells | A/D·W $(mol \cdot day^{-1} \cdot g^{-1})$ | D (day) | A (mol) | W (g) | Relative growth rate, μ $(day^{-1})$ | Content of the secondary metabolites (wt%) | Remarks |
|---|---|---|---|---|---|---|---|---|
| EX. 1 | 1 | $4.1 \times 10^{-8}$ | 4 | $9 \times 10^{-5}$ | 540 | 0.18(60) | 5.0 | |
| EX. 2 | 3 | $4.1 \times 10^{-8}$ | 12 | $3 \times 10^{-4}$ | 540 | 0.17(60) | 4.9 | |
| EX. 3 | 4.5 | $4.1 \times 10^{-8}$ | 18 | $4 \times 10^{-4}$ | 540 | 0.16(60) | 4.7 | |
| C.E.1 | 5.3 | $4.1 \times 10^{-8}$ | 21 | $5 \times 10^{-4}$ | 540 | 0.08(60) | 2.0 | |
| C.E.2 | 1 | $2.3 \times 10^{-5}$ | 4 | $5 \times 10^{-2}$ | 540 | 0.05(30) | 1.5 | Necrosis |
| C.E.3 | 0.08 | $5.4 \times 10^{-8}$ | 8hrs | $1 \times 10^{-5}$ | 540 | 0.10(30) | 2.5 | of the cells |
| C.E.4 | 1 | $4.5 \times 10^{-13}$ | 4 | $1 \times 10^{-9}$ | 540 | 0.11(60) | 5.0 | |

EX: Example

C.E.: Comparative Example

D: Feeding interval of the phytohormone

A: Amount of the phytohormone fed continuously to the medium

W: Fresh weight of the cells

## Example 4

The culture cells of Nicotiana tabacum were cultured in a 2 liter aerated culture vessel using Linsmaier & Skoog's medium. The volume of the liquid medium in the culture vessel during the culture was 1.8 liters. A liquid medium containing a nutrition substrate adjusted to a defined concentration was fed continuously (600 mℓ/day) to the culture vessel so that the concentration of the cells of Nicotiana tabacum becomes to be 211 g/ℓ as the fresh weight and at the same time a hormone component (4 × $10^{-5}$ mol of 2,4-dichlorophenoxy acetic acid) was added at 2 days interval (equal to the doubling time) while discharging the medium and the grown cells from the other end of the culture vessel to perform the culture. After the continuous culture was carried out for 60 days, the relative growth rate of the cells of Nicotiana tabacum was 0.38 (day $^{-1}$).

## Example 5

The culture cells of Rubia cordifolia were cultured in a 2 liter aerated culture vessel using Murashige & Skoog's medium. The volume of the liquid medium during the culture was 1.8 liters. A liquid medium containing a nutrition substrate adjusted to a defined concentration was fed continuously (600 mℓ/day) to the culture vessel so that the concentration of the cells of Rubia cordifolia becomes to be 138 g/ℓ as the fresh weight and at the same time a hormone component (1.1 × $10^{-5}$ mol of benzyl adenine and 1.1 × $10^{-6}$ mol of 2,4-dichlorophenoxy acetic acid) was added at 6 days interval (equal to the doubling time) while discharging the medium and the grown cells from the other end of the culture vessel to perform the culture. After the continuous culture was carried out for 60 days, the relative growth rate of the cells of Rubia cordifolia was 0.11 (day $^{-1}$) and the content of anthraquinones was 1.1%.

## Comparative Example 5

The procedure of Example 4 was repeated except that a hormone component (2.4 × $10^{-4}$ mol of 2,4-dichloro-phenoxy acetic acid) was added at 12 days interval (6 times of the doubling time). The relative growth rate of the cells of Nicotiana tabacum was 0.29 (day $^{-1}$).

## Comparative Example 6

The procedure of Example 5 was repeated except that a hormone component (1.1 × $10^{-6}$ mol of benzyl adenine and 1.1 × $10^{-7}$ mol of 2,4-dichlorophenoxy acetic acid) was added at 0.6 days interval (0.1 times of the doubling time). The relative growth rate of the cells of Rubia cordifolia was 0.05 (day $^{-1}$) and the content of anthraquinones was 0.7%.

Table 2  Culture of the plant cells other than Coptis japonica

| Plant | | D/doubling time of the cells | A/D·W $(mol \cdot day^{-1} \cdot g^{-1})$ | D (day) | A (mol) | W (g) | Relative growth rate, $\mu$ $(day^{-1})$ | Content of the secondary metabolites (wt%) |
|---|---|---|---|---|---|---|---|---|
| Nicotiana tabacum | EX. 4 | 1 | $5.3 \times 10^{-8}$ | 2 | $4 \times 10^{-5}$ | 380 | 0.38 | |
| | C.E.5 | 6 | $5.3 \times 10^{-8}$ | 12 | $2.4 \times 10^{-4}$ | 380 | 0.29 | |
| Rubia cordifolia | EX. 5 | 1 | $7.1 \times 10^{-9}$ | 6 | $1.1 \times 10^{-5}$ | 250 | 0.11 | 1.1 |
| | C.E.6 | 0.1 | $7.1 \times 10^{-9}$ | 0.6 | $1.1 \times 10^{-6}$ | 250 | 0.05 | 0.7 |

EX: Example

C.E.: Comparative Example.

## Claims

1. A method for the tissue culture of a plant using a liquid medium characterised in that a phytohormone is fed to the medium intermittently under the following conditions;

(a) the interval between successive feeds of the phytohormone is 0.5 to 5 times the doubling time of the cell, and

(b) the molar amount (A) of the phytohormone fed to the medium on each occasion satifies the equation

$$10^{-12} < \frac{A}{D \cdot W} < 10^{-6}$$

where W is the fresh weight of the cells present in the culture region (g) and D is the interval between successive feeds of the phytohormone (day).

2. A method according to Claim 1, wherein the phytohormone is fed to the medium when the residual concentration of the phytohormone in the medium has fallen to substantially zero.

3. A method according to Claim 1 or 2, wherein a liquid medium containing a nutrition substrate adjusted to a defined concentration is fed to one end of a culture region continuously or discontinuously and culture liquid is discharged from the other end of the culture region continuously or discontinuously, whereby culture is carried out under renewal of the medium in the culture region.

4. A method according to claim 1, 2 or 3 wherein the concentration of phytohormone in the medium immediately after each addition is from $10^{-4}$ to $10^{-10}$ M.

5. A method according to any one of the preceding claims wherein the cell concentration in the medium is maintained at 100 to 500 g/l.